# EUROPEAN PATENT APPLICATION

(11) **EP 4 799 600 A2**
(43) Date of publication of application: **02.09.2026**
(21) Application number: 24827769.1
(22) Date of filing: 24.10.2024
(51) Int. Cl.: A61K 8/02, A61K 8/64, A61K 8/65, A61K 8/67, A61K 8/73, A61K 8/85, A61K 8/86, A61K 8/92, A61Q 19/00, A61Q 19/08

(54) **COSMETIC PRODUCTS, METHOD OF PRODUCTION AND USE THEREOF**

(30) Priority: 25.10.2023 ES 202330876
(71) Applicant: Bioinicia, S.L., 46980 Paterna (Valencia) (ES); CONSEJO SUPERIOR DE INVESTIGACIONES CIENTIFICAS (CSIC), 28006 Madrid (ES)
(72) Inventor: LAGARON CABELLO, José María, 46980 Paterna (Valencia) (ES); PRIETO LÓPEZ, Cristina, 46980 Paterna (Valencia) (ES); TENO DÍAZ, Jorge, 46980 Paterna (Valencia) (ES); GONZÁLEZ ORTIZ, Danae, 46980 Paterna (Valencia) (ES)
(74) Representative: Pons IP
(86) International application number: PCT/ES2024/070653
(87) International publication number: WO 2025/088238

(57) **Abstract**

The present invention falls within the field of polymeric materials based on ultrafine fibres applied to the cosmetics sector, the invention relating to the method and application for manufacturing high-cosmetic-efficiency solid products in sheet format, manufactured by means of electro-hydrodynamic processing techniques, aero-hydrodynamic processing techniques, or any combination thereof.

## Description

### TECHNICAL SECTOR

The present invention falls within the area of polymeric materials based on ultrafine fibres applied to the cosmetics and pharmaceutical sector, the invention relating to its method and application for manufacturing high cosmetic efficiency solid products in film (layer or sheet) format manufactured by means of electro-hydrodynamic, aero-hydrodynamic processing techniques, or any combination thereof.

### BACKGROUND OF THE INVENTION

Cosmetic ingredients, also called bioactive compounds, are compounds comprising biomolecules, enzymes, liposomes, anti-inflammatory agents, probiotics, prebiotics, symbiotic agents, antioxidants, cell regenerators, anti-wrinkle, anti-glare, etc.

The formulation of conventional creams faces difficulties in combining various ingredients to achieve a stable, effective and safe composition. These difficulties include ingredient compatibility, appropriate emulsification, preservation, stability, texture, appearance, the bioactivity of active ingredients, penetration and absorption into the skin, allergies and irritation, as well as regulatory compliance. The use of solid creams in micrometre or sub-micrometre fibre format can solve several of these problems.

The formation of ultrafine fibres prepared by means of electro-hydrodynamic or aero-hydrodynamic processing, e.g. electrospinning, allows products to be designed for, for example, immediate and deep absorption of cosmetic bioactives through the skin or to absorb oil from the skin more efficiently. In the electrospinning technique, the effect of the voltage on the solution causes the solvent to evaporate rapidly, causing the fibres to form immediately and act as a cosmetic ingredient per se and/or efficiently trap the cosmetic ingredient in the fibres. In this latter case, the result is an optimal encapsulation of the cosmetic ingredient within the fibre structure, as well as a reduction in the ability of its molecules to crystallise, therefore, maintaining an amorphous or quasi-amorphous state, which facilitates its dissolution, diffusion and adsorption. The micro/nano structure of these materials provides a higher surface/volume ratio and better mechanical properties compared to other techniques to generate, for example, cosmetic films that disappear on the skin. Furthermore, it allows the preparation of cosmetic formulations in a solid state, without water or oils and potentially without any preservatives or other stabilising or emulsifying additives. By generating solid creams, it is also ensured that the water activity is very low and therefore no time-consuming tests would have to be carried out to ensure the absence of microbiological contamination. These techniques increase the sustainability of the cosmetic product, by reducing weight, volume and also allow packaging to be simplified. However, not all biopolymers can be processed using these techniques, or do not provide the required or desired characteristics of the cosmetic product in terms of cosmeticity, texture, appearance, bioactivity, penetration and absorption into the skin, adhesion to the skin, allergies and irritation, etc.

The present invention aims to solve the difficulties encountered in the state of the art by proposing cosmetic products with advantageous compositions that allow their optimal manufacture or processing using electro-hydrodynamic or aero-hydrodynamic techniques and present the optimal properties or characteristics required for their use as cosmetics, such as those mentioned in the previous paragraph.

### DESCRIPTION OF THE INVENTION

The present invention proposes a cosmetic product in sheet form (for example, in film, patch or mask format), configured to be applied to the skin, which may consist of a single layer (monolayer) or form a multilayer system, with the ability to release bioactive agents that are beneficial to the skin or to absorb oil or fat from the skin. The cosmetic product is manufactured using mainly electro-hydrodynamic, aero-hydrodynamic processing techniques or any combination thereof. Products can be manufactured in mono-axial, coaxial form, by co-deposition or layer-by-layer and optionally contains one or more cosmetic or bioactive ingredient(s) encapsulated therein. Each layer is composed of micrometre and/or sub-micrometre scale fibres.

The bioactive agent, if present, is encapsulated from solutions, dispersions or emulsions of the polymers and bioactives, which will form a solid product made of ultra-fine fibres that facilitate the homogeneous and controlled release of the bioactives onto the skin instantly.

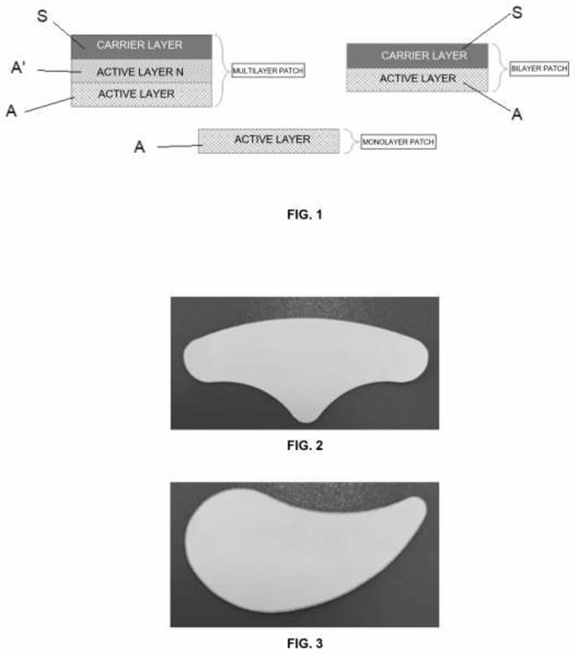

In a general manner, the products of the invention are composed of at least one layer of hydrophilic or hydrophobic polymeric fibres, which may be supported by a carrier layer also based on electrospun fibres, or a woven material, a non-woven material (also known as TNT), or by a continuous film, which can be transparent, coloured or opaque, as well as being a perforated film (Figure 1).

A first aspect of the present invention relates to a self-adhesive and highly skin-soluble cosmetic product based on essentially hydrophilic polymers, which may optionally contain and release bioactive agents into the skin, said product comprises or consists of:
a layer of polymeric fibres (A), wherein said layer optionally comprises at least one encapsulated bioactive agent, fibres have an average diameter size between 50 nm and 5 µm, more preferably between 100 nm and 3 µm, and even more preferably between 200 nm and 1 µm, measured using scanning electron microscopy (SEM); the fibre layer must have a surface density of at least 0.01 g/m², more preferably between 0.1 and 300 g/m², and even more preferably between 0.1 and 50 g/m² and wherein the polymeric fibres are formed by polymer mixtures selected from the list consisting of: pullulan/elastin; pullulan/polyethylene glycol (PEG), pullulan/elastin/collagen and polyethylene oxide (PEO)/polyethylene glycol.

The specific characteristics and composition of this cosmetic product allow it to adhere and dissolve quickly on moistened skin, providing an inherent effect of cosmetic firming, soothing/anti-irritation, anti-wrinkle and moisturising, which can be reinforced or supplemented by encapsulated bioactive agents that are released onto the skin to generate an optimal overall enhanced effect regarding anti-wrinkle, firming, soothing/anti-irritation, antibacterial, cleaning, nourishing, decongestant, antioxidant, depigmenting, regenerating, rejuvenating/anti-ageing, hydrating, emollient, illuminating, sebum-regulating, etc. In addition, by being made of very fine fibres, the large surface/volume ratio of these fibres generates a homogeneous application on the skin, copying its topology and thus facilitating its application, the immediate release of the bioactive agents by improving their adsorption on the skin and, therefore, its effectiveness.

Fibre is understood as elongated elements in which the length is greater than the width (diameter).

In a preferred embodiment, the polymeric fibres are formed by a pullulan/elastin mixture wherein the percentage by weight of elastin in the polymeric composition is between 3 and 8 %, more preferably between 3 and 5 % and even more preferably, 3 %, the remainder would be pullulan (up to 100 % by weight of the polymeric mixture).

In a preferred embodiment, the polymeric fibres are formed by a pullulan/PEG mixture wherein the percentage by weight of PEG in the polymeric composition is between 10 and 30 %, more preferably between 20 and 30 % and even more preferably, 25 %, the remainder would be pullulan (up to 100 % by weight of the polymeric mixture).

In a preferred embodiment, the polymeric fibres are formed by a pullulan/elastin/collagen mixture wherein the percentage by weight of elastin in the polymeric composition is between 3 and 8 %, that of collagen is between 10 and 50 %; more preferably the percentage of elastin in the polymeric composition is between 3 and 5 % and that of collagen is between 20 and 40 % and even more preferably, the percentage of collagen is 43.3 % and the percentage of elastin is 3.3 %, the remainder being pullulan (up to 100 % by weight of the polymeric mixture).

In a preferred embodiment, the polymeric fibres are formed by a PEO/PEG mixture wherein the percentage by weight of PEG in the polymeric composition is between 10 and 30 %, more preferably, between 20 and 30 %, even more preferably, 20 %, the remainder would be PEO (up to 100 % by weight of the polymeric mixture).

Preferably, in any of the aforementioned embodiments, elastin is of plant origin (plant-based elastin), more preferably, the elastin is hydrolysed wheat protein.

Preferably, in any of the aforementioned embodiments, the collagen is of non-animal origin such as collagen of microbial origin with or without genetic modification.

Preferably, in any of the aforementioned embodiments, the PEG is PEG 400.

Another aspect of the invention relates to a kit comprising the hydrophilic polymer-based cosmetic product described above and a water-based spray container (also called a mist) with or without other cosmetic, balsamic and/or odoriferous products. The spraying device allows the skin to be moistened before or after application of the cosmetic product so that the product adheres to the skin easily and dissolves on the surface thereof.

Another aspect of the invention also relates to a cosmetic product, similar to that described above in the first aspect of the invention, but based on hydrophobic polymers. In this case the cosmetic product would be non-soluble in the skin and would be used for the removal of oil or fat from the skin, preferably facial fat. Therefore, the present invention also relates to a cosmetic product for the absorption of oil or fat from the skin, also called anti-glare, comprising or consisting of:
a layer of polymeric fibres (B), wherein:
   said layer comprises an encapsulated bioactive, this bioactive agent being a fat or oil absorbing material (also called an anti-glare agent) wherein the polymeric fibres are formed by polymers selected from the list consisting of: polyhydroxyalkanoates (PHA), such as PHB, PHV, medium chain polyhydroxyalkanoates (mcl-PHA), and all the possible copolymers thereof such as PHBV among others, polylactic acid (PLA) and all its copolymers such as PGLA, poly-ε-caprolactone (PCL) and all the copolymers thereof such as PEG-PCL and PCLA, polyesters such as polybutylene succinate (PBS), polytrimethylene terephthalate (PTT), polybutylene terephthalate (PBT), and all the possible copolymers thereof such as poly(butylene adipate-co-terephthalate) (PBAT), polybutylene adipate succinate (PBAS), Poly(Butylene Adipate-co-Terephthalate) (PBAT), as well as any mixture of the above; or the polymeric fibres are formed by polymer mixtures selected from the list consisting of: PHA/PBS, PHA/PCL, PHA/PBAT or PHA/PBSA, the presence of a bioactive agent which is a fat-absorbing material being optional in this case and
wherein the polymeric fibres have an average diameter size between 50 nm and 5 µm, more preferably between 100 nm and 3 µm and, even more preferably, of between 200 nm and 1 µm, measured using scanning electron microscopy (SEM); the fibre layer must have a surface density of at least 0.01 g/m², more preferably between 0.1 and 500 g/m², even more preferably between 0.1 and 100 g/m².

In an even more preferred embodiment, the polymer forming the polymeric fibres of the layer (B) is selected from a PHA/PBS mixture, PHA/PCL, PHA/PBAT or PHA/PBSA, wherein the PHA content in the mixture is at least 30 % by weight of said mixture, so that the non-PHA polymer content is less than 70 % by weight in the mixture. These mixtures, given the characteristics of its composition, provide a more flexible and efficient cosmetic product by better absorbing oil or fat from the skin.

In an even more preferred embodiment, the polymer forming the polymeric fibres of the layer (B) is selected from a PHA/PBS mixture, PHA/PCL, PHA/PBAT or PHA/PBSA, wherein the non-PHA polymer content is less than 70 % by weight in the mixture and further comprises a bioactive agent which is a fat or oil absorbent material.

In a preferred embodiment, the fat-absorbent material, which may or may not have organomodifications to make the surface of the bioactive agent more compatible with the polymeric fibres and thus improve the dispersion and distribution thereof in the fibres, is selected from: carbonaceous compounds such as activated carbon, carbon black, graphite, graphene, carbon fibres or carbon tubes; silica, such as mesoporous silica, clays and nanoclays such as bentonites, kaolinites, vermiculites, laponites, sepiolite, or other porous minerals such as zeolites and other oil absorbers such as volcanic rock, as well as any of the combinations thereof. More preferably, the fat or oil absorbent material is selected from carbonaceous materials, mesoporous silica, nanoclays or any mixture thereof. Even more preferably, the fat or oil absorbent material is activated carbon.

The fat or oil absorbent material preferably has a micrometre or sub-micrometre particle size, e.g. nanometric, in other words, at least one of their dimensions (on average) is smaller than 1000 nm, measured by TEM (transmission electron microscopy) or SEM.

In a preferred embodiment, the content of fat or oil absorbent material in the polymeric fibre layer is less than 25 % by weight of the total weight of the layer, even more preferably less than 10 % and even more preferably less than 3 %. Preferably, at least, the content of the material in the layer is 0.001 % by weight.

In a preferred embodiment, the polymeric fibre layer (B) comprises the compound CTAB (hexadecyltrimethylammonium bromide) in an amount between 0.01 and 3 % by weight of the total weight of the polymeric fibre layer. This compound is optionally added during the method of preparation of polymeric fibre layer as a surfactant and improves the efficacy of the final product by enhancing the dispersion and distribution of the bioactive agent in the fibres and contributing to the absorption of fat or oil from the skin.

In the present invention, when reference is made to the "product of the invention", it includes both the product that is essentially based on hydrophilic polymers which serve in themselves to provide a cosmetic effect and which also optionally allow bioactive agents to be released for more effective absorption into the skin, as well as that which is based on hydrophobic polymers, which itself serves to remove fat or oil from the skin and which also optionally contain anti-glare additives to reinforce the effect of removing fat or oil from the skin, unless a particular product is specified.

In a preferred embodiment, the product of the invention comprises a carrier layer or substrate (S) on which the layer of polymeric fibres (A or B) is located, forming A-S or B-S multilayer structures. This carrier layer (S) consists of at least one layer of woven fibres, or woven non-woven (TNT) fibres or a continuous film of one or more hydrophilic and/or hydrophobic polymers. This substrate layer (S) must have a surface density of at least 0.1 g/m²; more preferably between 1 and 10,000 g/m²; even more preferably between 1 and 500 g/m².

In a preferred embodiment, the carrier layer (S) comprises or consists of a continuous polysaccharide based film, preferably the substrates comprise or consist of starch or cellulose such as cellulose acetate; or in woven or woven non-woven fabrics made of PCL or comprising or consisting of cellulose, or any combination thereof.

In the present invention, surface density, typically expressed in g/m² for each of the layers, is calculated by weighing a sample of known dimensions. Next, said weight is divided by the surface of the sample. This process is carried out with at least 5 samples of each layer in order to thus obtain a mean surface density value for the entire layer.

In the present invention, the term "encapsulation" relates to the highly scattered and distributed incorporation of the bioactive agent both inside the fibres of the polymers that make up each of the layers of the patch containing same, either by forming a separate *core-shell* (core-surface) phase, or by constituting a physical mixture with the polymeric material of the fibre, including what are known as solid solutions or dispersions; the bioactive agent therefore being able to be found both inside and on the surface of said fibres, or even in the interstitial spaces between them.

In a preferred embodiment, the self-adhesive cosmetic product, highly soluble and optionally allowing the release of bioactive agents into the skin that is essentially based on hydrophilic polymers, additionally comprises another layer of polymeric fibres (A'), forming an A-A' or A-A'-S layered structure, in case both are on a substrate (S). In this layer of polymeric fibres (A') the fibres have an average diameter size between 50 nm and 5 µm, more preferably between 100 nm and 3 µm, and even more preferably between 200 nm and 1 µm, measured using scanning electron microscopy (SEM); it has a surface density of at least 0.01 g/m², more preferably between 0.1 and 300 g/m², and even more preferably between 0.1 and 50 g/m² and the polymeric fibres are formed by polymer mixtures selected from the list consisting of: pullulan/elastin; pullulan/polyethylene glycol (PEG), pullulan/elastin/collagen, polyethylene oxide (PEO)/polyethylene glycol or by pullulan alone. The presence of bioactive agents in the A' layer is also optional and may carry the same or different bioactives as the A layer.

In a preferred embodiment, the cosmetic product based on hydrophobic polymers for the removal of fat or oil from the skin, additionally comprises another layer of polymeric fibres (B'), forming a B-B' or B-B'-S layered structure, in case both are on a substrate (S). In this layer of polymeric fibres (B') the fibres have an average diameter size between 50 nm and 5 µm, more preferably between 100 nm and 3 µm, and even more preferably between 200 nm and 1 µm, measured using scanning electron microscopy (SEM); the layer has a surface density of at least 0.01 g/m², more preferably between 0.1 and 500 g/m², and even more preferably between 0.1 and 100 g/m² and the polymeric fibres are formed by polymer mixtures selected from the list consisting of; polyhydroxyalkanoates (PHA), such as PHB, PHV, medium chain polyhydroxyalkanoates (mcl-PHA), and all the possible copolymers thereof such as PHBV among others, polylactic acid (PLA) and all its copolymers such as PGLA, poly-ε-caprolactone (PCL) and all the copolymers thereof such as PEG-PCL and PCLA, polyesters whether or not obtained from natural precursors such as polybutylene succinate (PBS), polytrimethylene terephthalate (PTT), polybutylene terephthalate (PBT), and all the possible copolymers thereof such as poly(butylene adipate-co-terephthalate) (PBAT), polybutylene adipate succinate (PBAS), Poly(Butylene Adipate-co-Terephthalate) (PBAT), as well as any mixture of the above. The presence of bioactive agents in the B' layer is optional and may carry the same or different bioactive agents as the B layer.

In a preferred embodiment, the product of the invention consists of a layer of polymeric fibres (layer A or B) that will come into contact with the skin and a substrate layer (layer (S)). They are multilayer structures: A-S or B-S

In another preferred embodiment, the product of the invention consists of a layer of polymeric fibres (layer A or B) which will come into contact with the skin, a second layer of polymeric fibres (A' or B' respectively) and a substrate layer (S) on which the previous layers are laid. They are multi-layered structures: A-A'-S or B-B'-S.

In the present invention the term "bioactive" refers to, without limitation, any natural or synthetic substance, beneficial for the skin, and more preferably for use in cosmetics.

The bioactive agents that may be used in the products of the present invention are selected without limitation from any cosmetic bioactive that performs any one or a combination of the following functions:
- Anti-wrinkle: such as peptides, botox-like bioactive agents (e.g. and limitation, botulinum toxin type A, spilanthes acmella also known as biobotox, dermatorelaxants such as some polypeptides (tri, tetra, penta, octapeptides), marine derivatives such as DMAE (dimethylaminoethanol), Tsubaki oil, Acetyl Hexapeptide-8, the famous Argireline (Acetyl Hexapeptide-3), or the improved version of the latter, known as SNAP-8), wakame seaweed extract, bakuchiol, etc.
- Firming agents: such as dimethylaminoethanol (DMAE), niacinamide (vitamin B3), collagen, elastin, etc.
- Soothing/Anti-irritation: such as Vitamin B12, cannabidiol (CBD), aloe vera extract, ceramides, etc.
- Antibacterial agents: such as azelaic acid, salicylic acid, malic acid, tea tree oil, etc,
- Cleaner: such as surfactants, betaine, coconut glucosides, saponins, etc.
- Nourishing: such as shea butter, almond oil, argan oil, vitamin E, etc.
- Decongestant: such as caffeine, guarana extract, ginseng extract, ginger extract, etc.
- Antioxidants: such as carotenoids, isoflavones, Vitamin C, phytosterols, etc.
- Depigmenting agents: such as glycolic acid, kojic acid, hydroxytyrosol, arbutin, etc.
- Regenerators: such as centella asiatica, epidermal growth factors, retinol, retinal, etc.
- Rejuvenating or anti-ageing: such as coenzyme Q10, jojoba oil, royal jelly, rosehip extract, etc.
- Moisturiser: such as hyaluronic acid, mucopolysaccharides, squalane, pro-vitamin B, etc.
- Emollient: such as oat extract, coconut oil, cucumber extract, sesame oil, etc.
- Illuminator: such as vitamin C, ferulic acid, lactic acid, mandelic acid, etc.
- Anti-glare: such as activated carbon, silica, clays such as bentonites, volcanic rock, etc.
- Sebo-regulator: azeloglycine, silicon, witch hazel extract, benzoyl peroxide, etc.
- Flavourings: essential oils such as lemon, tea tree, lavender, peppermint, rosemary, etc.

**In** another preferred embodiment, the bioactive agent used are of synthetic, natural, biotechnological origin such as, for example, by fermentation and/or recombinant or any mixture thereof.

**In** the present invention, the term "polymer" refers to macromolecular materials both in a pure ex-reactor state, and additives and post-processed materials in commercial formulas typically used by chemical industries, more commonly called plastic grades. Process additives may additionally be added to any of the polymers or plastic grades, biodegradability promoters or which confer stability, other filler-like additives, either in micro, sub-micron or nanometric form to improve their physico-chemical properties or their ability to retain and control the release of perfume. Such additives can be of the chemicals type, fibres, sheets or particles.

In another preferred embodiment, any of the layers of the product of the invention may contain aromatic substances or flavour enhancers.

The product of the invention may also contain some type of pigment or logo type printing, pictography, either multicolour or monocolour, as a differentiating element on the sides of the product. In this case the inks or pigments used are non-toxic, are biocompatible, with good organoleptic properties and are not detrimental to the integrity of the product or the encapsulated bioactive. Any type of printing or embossing can be used for this purpose as long as it is not detrimental to the integrity of the materials of the product, as well as the encapsulated bioactive. **It** may also have some kind of texture.

Moreover, the product of the invention can have any size and shape or flat motif, carried out by any conventional cutting method, either manual, using a die-cutting system, or laser cutting.

As regards the manufacture of the fibres that make up the layers of the product of the invention, these are preferably carried out by means of any of the known electro-hydrodynamic and aero-hydrodynamic techniques for obtaining fibres, more preferably using electrospinning,, direct beam printing using electro-hydrodynamic processing *(electrohydrodynamic direct writing),* melt electrospinning, solution blow spinning, or combination and/or variant thereof. Nevertheless, any other method for obtaining fibres may also be used, such as centrifugal jet spinning or the combination of this and those previously mentioned. Electro-hydrodynamic and aero-hydrodynamic techniques are based on the formation of polymeric micro- or submicrofibres at room temperature or lower, from a polymeric solution to which an electric field or gas pressure is applied. The fact that it is used in the form of a solution provides great versatility, since it enables various substances to be incorporated into the solution itself. At the same time, given that the processability thereof occurs at room temperature, it prevents certain problems such as the degradation of the bioactive agent.

In a preferred embodiment, the product of the invention is manufactured by means of electrospinning. In an even more preferred embodiment they are carried out by electrospinning using multi-output or multi-emitter injectors, whether made of needles or similar or made of porous materials. The advantage of these injectors over the so-called free-surface injectors is that they do not have a controlled output, also called *needleless electrospinning* or *free surface electrospinning,* is the greater control of fibre diameter dispersion and also of the homogeneity along the thickness. Control of fibre diameter dispersion facilitates reproducibility in release kinetics and thus cosmetic certification.

In a preferred embodiment the variation in fibre diameter is less than 35 %, in other words, that the fibre diameter variation is less than ±17.5 % above the average value. This value is measured by scanning electron microscopy (SEM).

In another preferred embodiment, fibre diameter variation for a given system with a multi-outlet injector is at least 15 % less than that which would occur with uncontrolled outlet injectors.

In another preferred embodiment, fibre diameter variation for a given system with a multi-outlet injector is at least 5 % less than that which would occur with uncontrolled outlet injectors.

With these techniques and the polymers mentioned above, in the present invention, the bioactive agent(s) is encapsulated such that the release can also be sustained. To carry out this encapsulation, techniques are used which include, without limitation: core-shell technology, co-deposition, surface modification electrospinning, electrospinning side-by-side, to generate Janus-type structures, direct mixing, emulsion techniques, pre-encapsulation in particles, or layer by layer deposition, etc.

In the present invention, core-shell technology is used in the case of electrospinning and solution blow spinning, using a concentrical nozzle through which the bioactive and/or nutraceutical agent in solution is delivered through the inner tube with or without polymers or simply polymers, while the encapsulating agent, in this case the polymer selected to prepare the corresponding layer, is passed through the outer tube. The use of nozzles with more than two concentrical tubes (triaxial or similar), can lead to further combinations of bioactive agent and polymer. Either way, this technology results in tubular fibres inside of which the bioactive agent and/or nutraceutical product(s) is (are) contained. In this case using non-water-soluble polymers, the molecules of the bioactive and/or nutraceutical agent diffuse through the fibre wall or through the internal porosity of the fibres, thus controlling the releasing process.

In the present invention, co-deposition consists of a deposition method in which two injectors simultaneously deposit, for example, on one hand, the polymeric solution with the bioactive agent and on the other hand another polymeric solution in which the bioactive agent is not soluble. This technique is used to control the release. It is also used by depositing two solutions containing a different bioactive agent, or which have different types of polymers, thus generating different fibre sizes and morphologies within the same membrane, and thus a different release profile of the bioactive agent(s). Co-deposition can be made of both the bioactive agent(s) and the encapsulant fibres, of particles and/or of fibres and/or of a mixture of the two can therefore be carried out. Furthermore, simultaneous electrospinning enables various properties to be combined within the same membrane.

In the present invention, the direct mixture can be, without limitation, of the encapsulated perfume and the encapsulating agent or of a suspension of particles containing the pre-encapsulated bioactive agent and the encapsulating agent, using monoaxial electrospinning, giving rise to cylindrical fibres in which the bioactive agent is embedded and dispersed within the fibre. Said mixture can be a homogeneous solution or a heterogeneous suspension.

In the present invention, the emulsion techniques refer to any emulsion of, without limitation, solvents or components, which give rise to encapsulation with several phases and which are processed by the processes known as electrohydrodynamic or aero-hydrodynamic emulsion processing. An emulsion is a dispersion of a liquid (dispersed phase) in the form of very small droplets within another liquid (continuous phase) with which it is generally immiscible. Emulsions can be direct, inverse or multiple. Direct emulsions are those in which the dispersed phase is a lipophilic substance and the continuous phase is hydrophilic. These emulsions are often called L/H or O/W. Inverse emulsions, on the other hand, are those in which the dispersed phase is a hydrophilic substance and the continuous phase is lipophilic. These emulsions are often called the abbreviation H/L or W/O. They can also be O/O emulsions, with two immiscible organic phases. Multiple emulsions are those in which, for example, the dispersed phase contains an inverse emulsion and the continuous phase is an aqueous liquid. Multiple emulsions can be H/L/H or W/O/W or O/W/O. Emulsions can also be formulated by means of the so-called Pickering emulsions that use particles to separate and stabilise the phases and by means of any other type of emulsion technology. In this way, the bioactive agent is encapsulated within the fibres in the organic phase, and the release also occurs in a controlled manner.

In the present invention, particle pre-encapsulation consists of obtaining fibres that are charged, or mixed in the event of co-deposition, with micro- or nanoparticles in which the bioactive agent has been previously encapsulated. For this, any encapsulation method is used that produces particles such as, and not limited to, *electrospray* air-assisted electrospray (EAPG), coacervation, emulsion-evaporation/emulsion-extraction, hot melt, interfacial polycondensation, complexing, gelling, fluidised bed, atomisation, lyophilisation, extrusion, *electrostatic droplet generation,* supercritical fluids, *TROMS,* etc., and mixtures thereof. These particles with bioactive and/or nutraceutical agents are typically added, in the case of direct mixing, to a solution of the selected polymer, such that, after any of the aforementioned processes, fibres with particles inside are obtained. In this case, the method of controlled release of the bioactive agent is carried out both by degradation of the particles and fibres, and by diffusion through the particles and the fibre, or it may even be the case where the two release mechanisms occur simultaneously.

In the present invention, the layer-by-layer deposition method consists of the use of a system in which the layers are deposited sequentially within the same process. In this regard, initially one of the layers is electrospun until reaching the desired thickness and then the second layer is electrospun on top of the first, obtaining a multilayer system *in situ.*

Therefore, another aspect of the invention relates to a method for obtaining any of the products of the invention (whether it is based on hydrophilic polymers or on hydrophobic polymers), wherein said method is based on the electrospinning technique comprising: preparation of a fibre layer (A or B) from a solution, suspension or emulsion of the polymer or polymer mixture to be formed into fibres where the polymer or polymer mixture is in a concentration of between 0.1 and 60 % by weight; the emitter voltage used is between 0.01 and 500kV and a collector voltage is between 0 kV and -500kV, with a flow rate of 0.0001 to 50,000 ml/h, at a temperature of between 1°C and 100°C, preferably between 20 and 40 °C, even more preferably at 40 °C; and a relative humidity preferably between 10 and 30 %, and even more preferably at 15 %.

Optionally, one or more bioactive agents are added to the solution, suspension or emulsion of the polymer or polymer mixture. Preferably, the bioactive agent(s) are at a total concentration between 0.01 and 70 % by weight in said solution, suspension or emulsion.

Preferably, a solution of the polymer or polymer mixture is prepared by choosing a solvent in which the polymers are soluble at the temperature at which the process is carried out. In the case of the preparation of mixtures of hydrophilic polymers (which is the case with the highly soluble, self-adhesive product with cosmetic effect on the skin), the preferred solvents are water and alcohols, such as methane, ethanol, isopropanol, butanol and trifluoroethanol, or any mixture thereof, more preferably, the solvent used is water. In the case of hydrophobic polymers (which is the case with the cosmetic product for the absorption of oil or fat from the skin), the preferred solvents are alcohols and organic solvents, and more preferably chloroform, DMF (dimethylformamide), HFIP (hexafluoropropanol), ethanol, methanol, butanol, acetone, trifluoroethanol or any mixture of the above.

When the polymer forming the fibres is not chemically compatible with the bioactive agent to be encapsulated (there is no physico-chemical interaction between them) or the bioactive agent has a low or very low solubility in the solvent(s) of the polymer required to carry out the electrospinning process, then any known emulsion route can be used to encapsulate the bioactive agent(s), instead of direct dissolution or suspension of the bioactive components. Solvents are preferably water, alcohols (such as methane, ethanol, isopropanol, butanol and trifluoroethanol, or any mixture thereof), organic solvents (such as chloroform), DMF (dimethylformamide), acetone or any combination thereof.

In a preferred embodiment, the layer of polymeric fibres (A or B) is prepared on a substrate layer (S), the substrate being as defined above.

In another preferred embodiment, a second layer of polymeric fibres (A' or B') is prepared on top of the first layer of fibres (A or B'), respectively) or on a substrate (S), wherein said layer (A' or B') is also prepared by electrospinning from a solution, suspension or emulsion of the polymer or polymer mixture to be formed into fibres where the polymer or polymer mixture is in a concentration of between 0.1 and 60 % by weight; the emitter voltage used is between 0.01 and 500kV and a collector voltage is between 0 kV and -500kV, with a flow rate of 0.0001 to 50,000 ml/h, at a temperature of between 1°C and 100°C, preferably between 20 and 40 °C, even more preferably at 40 °C; and a relative humidity preferably between 10 and 30 %, and even more preferably at 12 % and wherein the bioactive agent(s) are optionally added to the solution, suspension or emulsion at a total concentration between 0.01 and 70 % by weight.

A last aspect of the invention relates to the cosmetic use of the cosmetic product of the present invention based on essentially hydrophilic polymers to produce an inherently beneficial cosmeticising effect on the skin, as well as for the controlled release of one or more bioactive agent(s) into the skin, if the product contains bioactive agents, or for the absorption of oil or fat from the skin, if the product is the one based on essentially hydrophobic polymers.

The expression "inherently beneficial cosmeticising effect" implies that the cosmetic product itself based on essentially hydrophilic polymers, even without the presence of bioactive agents, is able to produce a beneficial cosmeticising effect on the skin. The "beneficial cosmeticising effect" refers to a firming, soothing, anti-irritation, anti-wrinkle and moisturising effect.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

### BRIEF DESCRIPTION OF THE FIGURES

**Fig. 1****.** Exemplary configurations of the product of the invention based on hydrophilic polymers according to monolayer or multilayer systems.
**Fig. 2****.** Product of Example 1
**Fig. 3****.** Product of Example 3
**Fig. 4****.** Graphs showing caffeine absorption through synthetic membrane (Strat-M) simulating human skin versus the absorption of the same amount of caffeine from a cream simulant with the same composition (left); and caffeine absorption through human skin from the caffeine patch (right) described in example 9.
**Fig. 5** Product of Example 7, where it can be seen that the patch absorbs oil.

### EXAMPLES

Next, the invention will be illustrated by means of assays carried out by the inventors that demonstrate the effectiveness of the product of the invention.

The polymers used in the examples for the preparation of the cosmetic products have been purchased commercially, as set out below:
Pullulan: CAS No.: 9057-02-7; PEG400: CAS No. 25322-68-3; Hydrolysed wheat protein (plant-based elastin) CAS No.: 222400-28-4 or 70084-87-6; Collagen of recombinant origin CAS No.: 9007-34-5; PEO: CAS No.: 25322-68-3.

### Example 1: Bilayer product format with antioxidant effect (Figure 2), using the formula pullulan/PEG, in which the carrier layer is a continuous starch film.

On the starch film (100 µm thick) placed on a rotating collector (200 rpm) the hydrophilic fibres with encapsulated bioactive agent were deposited. Said manufacture was carried out at a temperature of 40°C and a relative humidity of 15%. To do so, the starting point was a solution of pullulan at 11.2 % by weight (wt.%), 3.75 % polyethylene glycol (PEG400) and 0.076 % hydroxytyrosol, commercially called Olivan Antiox, in deionised water. In this polymeric solution the polymer:bioactive agent ratio was 99.5:0.5. Once both components were dissolved, the fibre sheet was then manufactured using the electrospinning technique in a 5-needle linear multi-output injector. An emitter voltage of 25kV was used to produce the fibre mesh, as well as a collector voltage of -25kV. A flow rate of 5 ml/h was also used. The fibres were deposited on a rotating collector (200 rpm). In this case the surface density is 5 g/m². This product was cut to a specific shape for application to the cheek or forehead, as can be seen in figure 2. In this type of patch the layer with the bioactive agent is solubilised in the partially wet skin.

### Example 2: Bilayer product format with firming effect, with the formulation pullulan/elastin in which the carrier layer is a cellulose acetate film.

On the cellulose acetate film (25 µm thick) placed on a rotating collector (200 rpm) the hydrophilic fibres with encapsulated bioactive agent were deposited. Said manufacture was carried out at a temperature of 30°C and a relative humidity of 20%. To do so, the starting point was a solution of pullulan at 14.55 % by weight (wt.%), 0.45 % hydrolysed wheat protein (plant-based elastin), 0.625 % Resveratrol and Biotin Liposomes (Carrivect 7), and 0.048 % palmitoyl pentapeptide-4 and colloidal gold commercially called Matrigold, in deionised water. In this polymeric solution the polymer:bioactive agent ratio was 95.7:4.3. Once both components were dissolved, the fibre sheet was then manufactured using the electrospinning technique in a 5-needle linear multi-output injector. An emitter voltage of 35kV was used to produce the fibre mesh, as well as a collector voltage of - 25kV. A flow rate of 5 ml/h was also used. The fibres were deposited on a rotating collector (200 rpm). In this case the surface density is 5 g/m². In this type of patch the layer with the bioactive agent is solubilised in the partially wet skin.

### Example 3: Three-layer product format with moisturising effect, using the formula pullulan/PEG, in which the carrier layer is a cellulose non-woven fabric. (Figure 3)

On an 80 g/m²cellulose non-woven fabric, placed on a rotating collector (200 rpm) the hydrophilic fibres with encapsulated bioactive agent were deposited. Said manufacture was carried out at a temperature of 30°C and a relative humidity of 20%.

In this case a first layer was deposited, the starting point was a solution of pullulan at 11.25 % by weight (wt.%), 3.75 % polyethylene glycol (PEG400) and 0.474 % liposomes with moisturising effect Matrigold and lubricant (Carrivect 1), and 0.316 % Niacinamide and vitamin blend commercially called Beauplex VH-DSM NP, in deionised water. In this polymeric solution the polymer:bioactive ratio was 95:5. Once both components were dissolved, the fibre sheet was then manufactured using the electrospinning technique in a 5-needle linear multi-output injector. An emitter voltage of 30kV was used to produce the fibre mesh, as well as a collector voltage of -25kV. A flow rate of 5 ml/h was also used. The fibres were deposited on a rotating collector (200 rpm). In this case the surface density is 5 g/m².

On top of the previous layer another layer of fibres was deposited starting from a solution of pullulan at 11.25 % by weight (wt.%), 3.75 % polyethylene glycol (PEG400) in deionised water. In this polymeric solution the ratio between the polymers is 75:25. Once both components were dissolved, the fibre sheet was then manufactured using the electrospinning technique in a 5-needle linear multi-output injector. An emitter voltage of 25kV was used to produce the fibre mesh, as well as a collector voltage of -25kV. A flow rate of 5 ml/h was also used. The fibres were deposited on a rotating collector (200 rpm) on top of the previous layer. In this case the surface density is 1 g/m².

This product was cut to a specific shape for application to the dark circles (skin under the eyes) in figure 3. In this type of patch the water-soluble layers are solubilised in the partially wet skin.

### Example 4: Three-layer product format with Vitamin C, using the formula pullulan/PEG, wherein the carrier layer is a cellulose acetate film

On the cellulose acetate film (25 µm thick) placed on a rotating collector (200 rpm) the hydrophilic fibres with encapsulated bioactive agent were deposited. Said manufacture was carried out at a temperature of 30°C and a relative humidity of 20%.

In this case a first layer was deposited, the starting point was a solution of pullulan at 11.2 % by weight (wt.%), 3.75 % polyethylene glycol (PEG400) and 0.625 % Resveratrol and Biotin liposomes (Carrivect 7), and 0.08 % of gold microparticles with vitamin C commercially called Golden C in deionised water. In this polymeric solution the polymer:bioactive ratio was 95.5:4.5. Once both components were dissolved, the fibre sheet was then manufactured using the electrospinning technique in a 5-needle linear multi-output injector. An emitter voltage of 35kV was used to produce the fibre mesh, as well as a collector voltage of -25kV. The fibres were deposited on a rotating collector (200 rpm). In this case the surface density is 5 g/m²

On top of the previous layer another layer of fibres was deposited starting from a solution of pullulan at 11.2 % by weight (wt.%), 3.75 % polyethylene glycol (PEG400) in deionised water. In this polymeric solution the ratio between the polymers is 75:25. Once both components were dissolved, the fibre sheet was then manufactured using the electrospinning technique in a 5-needle linear multi-output injector. An emitter voltage of 25kV was used to produce the fibre mat, as well as a collector voltage of -25kV. A flow rate of 5 ml/h was also used. The fibres were deposited on a rotating collector (200 rpm). In this case the surface density is 1 g/m². In this type of patch the water-soluble layers are solubilised in the partially wet skin.

### Example 5: Three-layer product format with Collagen and Hyaluronic Acid, using the pullulan/elastin/collagen formula, in which the carrier layer is a starch film

On a starch film (100 µm thick) placed on a rotating collector (200 rpm) the hydrophilic fibres with encapsulated bioactive agent were deposited. Said manufacture was carried out at a temperature of 40°C and a relative humidity of 15%.

In this case a first layer was deposited, the starting point being a solution of pullulan at 8 %, hydrolysed wheat protein (plant-based elastin) at 0.5 % and 6.5 % collagen of recombinant origin by weight (wt.%) in deionised water. Once both components were dissolved, the fibre sheet was then manufactured using the electrospinning technique in a 5-needle linear multi-output injector. An emitter voltage of 35kV was used to produce the fibre mesh, as well as a collector voltage of -30kV. A flow rate of 5 ml/h was also used. The fibres were deposited on a rotating collector (200 rpm). In this case the surface density is 5 g/m²

On top of the previous layer, another layer of fibres was deposited starting from a solution of 14.4 % pullulan and 0.8 % hyaluronic acid by weight (wt.%), in a 90:10 mixture of deionised water/isopropanol. Once both components were dissolved, the fibre sheet was then manufactured using the electrospinning technique in a 5-needle linear multi-output injector. An emitter voltage of 25kV was used to produce the fibre mesh, as well as a collector voltage of -20kV. A flow rate of 5 ml/h was also used. The fibres were deposited on a rotating collector (200 rpm). In this case the surface density is 5 g/m². In this type of patch the electrospun water-soluble layers are solubilised in the partially moist skin.

### Example 6: Bilayer product format with caffeine, using the pullulan/PEG formula and in which the carrier layer is a layer of electrospun PCL fibres.

In this product, a layer of electrospun fibres was used as the outer or carrier layer. To do so, a solution of poly-ε-caprolactone (PCL) at 12% by weight, in 79% by weight of chloroform and 9% methanol is made. For producing this layer, an emitter voltage of 23kV and a collector voltage of -1kV were used, a flow rate of 10 ml/h was also used, through a multi-outlet linear injector. This very last layer must have a surface density between 12 g/m² since the main function thereof is to protect and carry the product. The hydrophilic fibres described below were deposited on this layer.

On top of the previous layer a layer of fibres was deposited starting from a solution of pullulan at 9 % by weight (wt.%), 2.25 % PEG400, 2.3 % caffeine, and 1.2 % oleic acid, in water/isopropanol in a 92:8 ratio. In this polymeric solution the polymer:caffeine ratio was 80:20. Once both components were dissolved, the fibre sheet was then manufactured using the electrospinning technique in a 5-needle linear multi-output injector. An emitter voltage of 25kV was used to produce the fibre mat, as well as a collector voltage of -15kV. A flow rate of 5 ml/h was also used. The fibres were deposited on a rotating collector (200 rpm). In this case the surface density is 20 g/m². It was processed at 30 °C and 20 % relative humidity.

Once manufactured, the layers are bonded together using the calendering technique at a speed of 2.56 rpm and heating only the roller that is in contact with the outer layer (PCL) to 40 °C. In this way, adhesion between the layers is ensured, and a coalescence of the fibres, reduction of porosity, of the outer layer of PCL is carried out.

In this type of patch the layer with the bioactive agent is solubilised in the partially wet skin.

### Example 7: Hydrophobic monolayer product format with anti-glare properties.

For the manufacture of this product, the starting point was a solution of poly-ε-caprolactone (PCL) at 5 % by weight, Ethyl cellulose at 5 % by weight as an additive, and 0.3 % activated carbon in chloroform/methanol at a 90:10 ratio by weight. For the production, an emitter voltage of 23kV and a collector voltage of -10kV were used, a flow rate of 10 ml/h was also used, through a multi-outlet linear injector. This last layer had a surface density of 12 g/m².

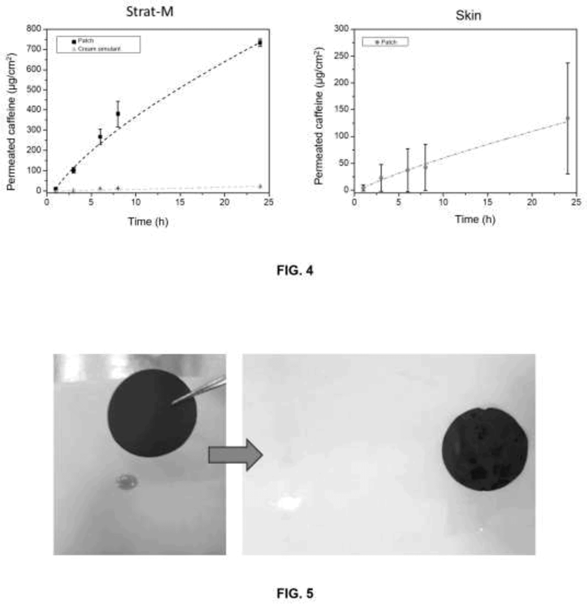

This layer is not solubilised by skin moisture, its sole function is to absorb fat from the skin, thus eliminating the facial glare generated by the excess thereof. Figure 5 shows the oil absorption capacity of said patch as an example.

### Example 8: Bilayer product format with caffeine using the pullulan/PEG formulation and hyaluronic acid (HA) using the pullulan formulation produced by monoaxial co-electrospinning and the carrier layer is a starch film.

On a starch film (100 µm thick), placed in a *roll to roll* system, the substrate is allowed to pass under both injectors continuously, i.e. pullulan/HA and pullulan/PEG/caffeine depositions are simultaneously made. Said manufacture was carried out at a temperature of 30°C and a relative humidity of 20%, on a Fluidnatek LE-500 unit from Bioinicia S.L.

In this case, two different solutions were simultaneously deposited. For this purpose, a solution was used with pullulan at 9 % by weight (wt.%), 2.25 % PEG400, 2.3 % caffeine, and 1.2 % oleic acid, in water/isopropanol in a 92:8 ratio. In this polymeric solution the polymer:caffeine ratio was 80:20. Simultaneously, a solution of pullulan at 14.4 % and 0.8 % hyaluronic acid by weight (wt.%) was used. Both solutions were simultaneously deposited, achieving a combination of pullulan/HA and pullulan/PEG/caffeine fibres in a layer with a surface density of 10 g/m². In this type of patch the layer with the bioactive agent is solubilised in the partially wet skin.

### Example 9: Bilayer product format using the PEO/PEG formula and containing caffeine in which the carrier layer is a layer of electrospun PCL fibres.

As in previous examples, a layer of electrospun fibres was used as the outer or carrier layer in this product. To do so, a solution of poly-ε-caprolactone (PCL) at 12% by weight, in 79% by weight of chloroform and 9% methanol is made. For producing this layer, an emitter voltage of 23kV and a collector voltage of -1kV were used, a flow rate of 10 ml/h was also used, through a multi-outlet linear injector. This last layer had a surface density of 12 g/m² since the main function thereof is to protect and carry the product. The hydrophilic fibres described below were deposited on this layer.

On top of the previous layer, a layer of fibres was deposited starting from a solution of PEO at 9 % by weight (wt.%), 2.25 % PEG400, 3.2 % caffeine, and 1.6 % oleic acid, in Chloroform/Methanol at an 80:20 ratio. In this polymeric solution the polymer:caffeine ratio was 80:20. Once both components were dissolved, the fibre sheet was then manufactured using the electrospinning technique in a 5-needle linear multi-output injector. An emitter voltage of 20kV was used to produce the fibre mesh, as well as a collector voltage of - 5kV. A flow rate of 10 ml/h was also used. The fibres were deposited on a rotating collector (200 rpm). In this case the surface density is 73 g/m². It was processed at 30 °C and 20 % relative humidity.

As in example 6, the different layers are bonded together using the calendering technique at a speed of 2.56 rpm and heating only the roller that is in contact with the outer layer (PCL) to 40 °C. In this way, adhesion between the layers is ensured, and a coalescence of the fibres, reduction of porosity, of the outer layer of PCL is carried out.

In this type of patch the layer with the bioactive agent is solubilised in the partially wet skin.

This patch was evaluated by a caffeine permeation study using a Franz cell system. For this purpose, 1 cm²patches were used, on a synthetic membrane (Strat-M) and on human skin. A pH buffer of 7.4 at 37 °C under stirring was used in the receiving compartment. Each experiment was conducted in triplicate. Figure 4 shows the caffeine permeation graphs on synthetic membrane (Strat-M), both as a patch (square) and as a cream simulant with the same composition as the patch (triangle), where it can be observed that with a patch format the permeation of caffeine is higher than in liquid. Moreover, it also shows the graph of caffeine permeation into the skin which justifies that the patch proposed in this patent facilitates the permeation of bioactives, as in this case caffeine.

### Example 10: Bilayer product format with antioxidant effect, manufactured from core-shell fibres using the pullulan and pullulan/elastin/collagen formulas using a non-woven cellulose fabric as a carrier layer.

On an 80 g/m²cellulose non-woven fabric, the hydrophilic fibres with encapsulated bioactive agent were deposited on a rotating collector (200 rpm). Said manufacture was carried out at a temperature of 30°C and a relative humidity of 20%.

In this case, the fibre layer was deposited, using a device made up of coaxial nozzles. A solution of pullulan at 12 % by weight (%wt) in deionised water is injected through the external nozzle (Shell). A solution of pullulan at 8 % is injected through the internal nozzle (Core), Hydrolysed wheat protein (plant-based elastin) at 0.5% and 6.5% by weight of recombinant collagen in deionised water. This results in the formation of tubular pullulan fibres such that collagen is encapsulated by the same. For said production, an emitter voltage of 20kV, a collector voltage of -10Kv, and a flow rate of 10 ml/h were used. In this case the surface density is 10 g/m². In this type of patch the layer with the bioactive agent is solubilised in the partially wet skin.

### Example 11: Three-layer product format, with collagen and Eucalyptus scent, wherein the fibre interlayer is produced by emulsion-electrospinning based on the pullulan formula and the carrier layer is a cellulose non-woven fabric.

On an 80 g/m²cellulose non-woven fabric, the hydrophilic fibres with encapsulated bioactive agent were deposited on a rotating collector (200 rpm). Said manufacture was carried out at a temperature of 30°C and a relative humidity of 20%.

In this case a first layer was deposited, the starting point being a solution of pullulan at 13 % by weight in water and adding the eucalyptus essential oil with 3 % Span20 surfactant little by little to obtain an emulsion with a 5:1 polymer: essential oil ratio. The system was homogenised using an UltraTurrax T-25 homogeniser (IKA, Staufen, Germany) at 17,000 rpm for 5 min, followed by 5 minutes of ultrasound (Bandelin Sonopuls, Berlin, Germany) and processed by means of electrospinning. To do this, an emitter voltage of 20kV and a collector voltage of -10kV were used; a flow rate of 5 ml/h was also used, through a 22G multi-needle linear injector. The fibres were deposited on a rotating collector (200 rpm). This layer has adhesive layer surface density of 2 g/m².

On top of the previous layer another layer of fibres was deposited starting from a solution of pullulan at 8 % by weight (wt.%), 2 % Collagen in deionised water. Once both components were dissolved, the fibre sheet was then manufactured using the electrospinning technique in a 5-needle linear multi-output injector. An emitter voltage of 25kV was used to produce the fibre mat, as well as a collector voltage of -25kV. A flow rate of 5 ml/h was also used. The fibres were deposited on a rotating collector (200 rpm) on top of the previous layer. In this case the surface density is 5 g/m². In this type of patch the water-soluble layers are solubilised in the partially wet skin.

## Claims

1. A self-adhesive and soluble cosmetic product to be applied to the skin, wherein the product comprises:
a layer of polymeric fibres (A), wherein the fibres have an average diameter size between 50 nm and 5 µm; and are formed by polymer mixtures selected from the list consisting of: pullulan/elastin; pullulan/polyethylene glycol (PEG), pullulan/elastin/collagen and polyethylene oxide (PEO)/polyethylene glycol and wherein the layer (A) has a surface density of at least 0.01 g/m².

2. The cosmetic product, according to claim 1, wherein said layer optionally comprises at least one encapsulated bioactive agent.

3. The cosmetic product, according to claim 1 or 2, wherein the polymeric fibres are formed by a pullulan/elastin mixture and the percentage by weight of elastin in the polymeric mixture is between 3 and 8 %, the remainder would correspond to pullulan.

4. The cosmetic product, according to claim 1 or 2, wherein the polymeric fibres are formed by a pullulan/PEG mixture and the percentage by weight of PEG in the polymeric mixture is between 10 and 30 % and the rest would correspond to pullulan.

5. The cosmetic product, according to claim 1 or 2, wherein the polymeric fibres are formed by a pullulan/elastin/collagen mixture and the percentage by weight of elastin in the polymeric mixture is between 3 and 8 %, that of collagen being between 10 and 50 %, the rest would correspond to pullulan.

6. The cosmetic product, according to claim 1 or 2, wherein the polymeric fibres are formed by a PEO/PEG mixture and the percentage by weight of PEG in the polymeric mixture is between 10 and 30 % and the rest would correspond to PEO.

7. The cosmetic product, according to any of claims 1 to 3 or 5, wherein the elastin is a plant-based elastin.

8. The cosmetic product, according to claim 7, wherein the plant-based elastin is hydrolysed wheat protein.

9. The cosmetic product, according to any one of claims 1, 2, 5, 7 or 8, wherein the collagen is of non-animal origin.

10. The cosmetic product for the absorption of oil or fat from the skin, comprising or consisting of:
a layer of polymeric fibres (B), wherein:
said layer comprises a bioactive agent, this bioactive agent being a fat or oil absorbing material, and wherein the polymeric fibres are formed by polymers selected from the list consisting of: polyhydroxyalkanoates (PHA), medium chain polyhydroxyalkanoates (mcl-PHA), polylactic acid (PLA), poly-ε-caprolactone (PCL), polybutylene succinate (PBS), polytrimethylene terephthalate (PTT), polybutylene terephthalate (PBT), copolymers of any of the foregoing, and any of the combinations thereof,
or
wherein said layer optionally comprises a bioactive agent, this bioactive agent being a fat or oil absorbing material, and wherein the polymeric fibres are formed by polymer mixtures selected from the list consisting of: PHA/PBS, PHA/PCL, PHA/PBAT and PHA/PBSA, and
wherein the polymeric fibres have an average diameter size between 50 nm and 5 µm and the fibre layer has a surface density of at least 0.01 g/m².

11. The cosmetic product, according to claim 10, wherein the polymer forming the polymeric fibres of the layer (B) is selected from a PHA/PBS, PHA/PCL, PHA/PBAT and PHA/PBSA mixture, and wherein the PHA content in the mixture is at least 30 % by weight of the polymeric mixture.

12. The cosmetic product, according to claim 10 or 11, comprising a fat or oil absorbent material selected from: carbonaceous compounds, selected from activated carbon, carbon black, graphite, graphene, carbon fibres or carbon tubes; silica, clays, selected from bentonites, kaolinites, vermiculites, laponites, sepiolite, zeolites, volcanic rock, as well as any of the combinations thereof.

13. The cosmetic product, according to any of claims 10 to 12, wherein the content of fat or oil absorbent material in the polymeric fibre layer (B) is less than 25 % by weight of the total weight of the layer.

14. The cosmetic product, according to any of claims 10 to 13, wherein the polymeric fibre layer (B) comprises hexadecyltrimethylammonium bromide in an amount between 0.01 and 3 % by weight of the total weight of the polymeric fibre layer (B).

15. The cosmetic product, according to any of the preceding claims, comprising a carrier or substrate layer (S) on which the layer of polymeric fibres (A) or (B) is located.

16. The cosmetic product, according to claim 15, wherein the carrier or substrate layer (S) has a surface density of at least 0.1 g/m².

17. The cosmetic product according to any of claims 15 or 16, wherein the carrier or substrate layer (S) comprises or consists of starch, cellulose, cellulose acetate, polycaprolactone (PCL) or any combination thereof.

18. **The** cosmetic product according to any of the preceding claims, wherein the fibre layers (A) or (B) are obtained using electro-hydrodynamic, aero-hydrodynamics techniques or combinations thereof.

19. **The** cosmetic product according to claim 18, wherein the fibre layers (A) or (B) are obtained using *electrospinning.*

20. A method of production of the cosmetic product defined in any of claims 1 to 19, wherein said method is based on the electrospinning technique comprising:
preparation of a fibre layer (A or B) from a solution, suspension or emulsion of the polymer or polymer mixture that is forming the fibres, wherein the polymer or polymer mixture is at a concentration between 0.1 and 60 % by weight and the process conditions are as follows: the emitter voltage used is between 0.01 and 500 kV and a collector voltage between 0 kV and -500 kV; a flow rate of 0.0001 to 50,000 ml/h, a temperature between 1 °C and 100 °C, and a relative humidity between 10 and 30 %.

21. **The** method, according to claim 20, wherein one or more bioactive agents are added to the solution, suspension or emulsion of the polymer or polymer mixture at a total concentration between 0.01 and 70 % by weight in said solution, suspension or emulsion.

22. **The** method, according to claim 20 or 21, wherein the fibre layer (A) or (B) is prepared on a substrate layer (S).

23. **The** method, according to any of claims 20 to 22, wherein controlled outlet, multi-output or multi-transmitter injectors are used.

24. **The** method, according to claim 23, wherein the variation of the fibre diameter is less than 35 %.

25. A cosmetic use of the cosmetic product defined in any of claims 1 to 9, 15 to 19 for producing a beneficial cosmeticising effect on the skin.

26. A use of the product defined in any of claims 2 to 9, 15 to 19, for the controlled release of one or more bioactive agent(s) into the skin in case the cosmetic product comprises at least one bioactive agent.

27. A cosmetic use of the cosmetic product defined in any of claims 9 to 19 for the absorption of oil or fat from the skin.

28. A kit comprising a cosmetic product, as described in claims 1 to 9 and 15 to 19 and a water-based spray container with or without other cosmetic, balsamic and/or odoriferous products.
